# EUROPÄISCHE PATENTSCHRIFT

(11) **EP 1 551 490 B1**
(45) Veröffentlichungstag und Bekanntmachung des Hinweises auf die Patenterteilung: **02.05.2007**
(21) Anmeldenummer: 03808651.8
(22) Anmeldetag: 19.08.2003
(51) Int. Cl.: A61M 25/01

(54) **FÜHRUNGSEINRICHTUNG INSBESONDERE ZUM POSITIONIEREN VON KATHETERN IN EINEM KÖRPERGANG**
GUIDE DEVICE IN PARTICULAR FOR POSITIONING CATHETERS IN A BODY DUCT
DISPOSITIF DE GUIDAGE NOTAMMENT POUR LA MISE EN PLACE DE CATHETERS DANS UN CANAL CORPOREL

(30) Priorität: 15.10.2002 CH 171202; 20.06.2003 CH 108303
(43) Veröffentlichungstag der Anmeldung: 13.07.2005
(73) Patentinhaber: Von Weymarn-Schärli, Alexander, 4059 Basel (CH)
(72) Erfinder: Von Weymarn-Schärli, Alexander, 4059 Basel (CH)
(74) Vertreter: Volpert, Marcus
(86) Internationale Anmeldenummer: PCT/CH2003/000556
(87) Internationale Veröffentlichungsnummer: WO 2004/035124

(56) Entgegenhaltungen:
- EP-A- 0 415 332
- WO-A-96/21488
- US-A- 5 337 733
- US-A- 5 345 937
- US-A- 5 706 827

## Beschreibung

Die Erfindung bezieht sich auf eine Führungseinrichtung, insbesondere zum Positionieren von Kathetern in einem Körpergang, nach dem Oberbegriff des Patentanspruchs 1.

In der WO 02/34324 A2 ist das Problem angesprochen, bei Führungsdrähten einen Abschnitt aus einem superelastischen Material mit einem Abschnitt aus Stahl zu verbinden, welcher wesentlich steifer als der erstgenannte Abschnitt ist. Der aus der WO 00/40288 A1 bekannte Führungsdraht hat an seinem distalen Abschnitt einen formbaren Körper, dessen Flexibilität fortlaufend verändert ist und seine formbare Eigenschaft beibehalten soll.

In der EP 0 714 315 B1 ist das Problem angesprochen, dass der Führungsdraht während des Zurückziehens eines Katheters dazu neigt, verschoben zu werden. Der hier offenbarte Führungsdraht ist derart ausgestaltet, dass seine Verschiebung visuell deutlich erkennbar ist.

Der in der DE 200 19 484 U1 beschriebene Führungsdraht weist in regelmässigen Abständen einen mit minimalen Mengen ferromagnetischer Partikel dotierten, nichtferromagnetischen Grundkörper auf, wobei der Führungsdraht aus einem zylindrischen Hohlschlauch aus nichtferromagnetischem Material, wie zum Beispiel Kunststoff, gebildet ist, der einen zylindrischen Träger enthält, welcher in regelmässigen Abständen mit Haufen aus ferromagnetischen Partikeln markiert ist. Dieser Führungsdraht ist insbesondere für magnetresonanztomografisch gesteuerte Verfahren geeignet.

Eine Filtervorrichtung zum Fangen von embolischem Material in einem Blutgefäss, welche Filtervorrichtung auch einen Führungsdraht umfasst, ist aus der DE/EP 0 980 278 T1 bekannt. Aus der letztgenannten Druckschrift ist ferner ein Katheter bekannt, welcher einen spreizbaren Stent, wobei der Stent einen ersten Durchmesser sowie einen zweiten gespreizten Durchmesser aufweist, und auch einen Spreizrahmen hat, der zwischen einem kontrahierten und einem aufgeweiteten Zustand spreizbar ist. Ferner hat der Katheter eine Hülle, wobei während des Gebrauchs die Hülle zurückgezogen wird, um den Spreizrahmen und den Stent freizugeben. Der Spreizrahmen kann eine Aufpumpdichtung aufweisen. Der Katheter hat ferner ein Aufpumpsystem, das ein zur Aufnahme von Druckfluid ausgelegtes erstes Lumen und ein zum Evakuieren von Gas ausgelegtes zweites Lumen aufweist. Die Aufpumpdichtung hat ferner eine Eingangsöffnung in Fluidverbindung mit dem ersten Lumen des Aufpumpsystems und eine Ausgangsöffnung in Fluidverbindung mit dem zweiten Lumen des Aufpumpsystems. Die Aufpumpdichtung kann einen schlauchförmigen Ballon aufweisen.

In der EP 0 826 389 B1 ist ein Führungsdraht mit einem Kerndraht beschrieben, welcher eine Mehrzahl dünner, miteinander verdrillter Adern umfasst. Der Kerndraht hat einen halbkreisförmigen Querschnitt und schafft dadurch Raum für einen Kanal für einen Fluiddurchtritt. Führungsdraht und Kerndraht sind Teil eines Katheters, dessen vorderes Ende offen ist und dadurch einen Fluidaustritt in Längsrichtung des Katheters ermöglicht.

Aus der EP 0 823 261 A2 ist ein Führungsdraht bekannt, welcher ein äusseres Rohr aufweist, das aus einem flexiblen Material ohne Rückstellkraft gebildet ist. Ferner hat der Führungsdraht einen inneren Kerndraht, welcher in dem äusseren Rohr hin- und herbewegbar ist. Der Kerndraht hat ein distales Ende, das auf der einen Seite zwar flexibel, auf der anderen Seite jedoch in einer bestimmten Weise derart eine Rückstellkraft aufweist, dass das distale Ende, sofern möglich, insbesondere am distalen Ende des äusseren Rohres in vorbestimmter Weise gekrümmt absteht. Dazu ist das distale Ende des äusseren Rohres sehr flexibel ausgebildet.

In der EP 0 778 044 A2 ist eine Führungsdraht-Einheit beschrieben, bei welcher eine Sondeneinheit in eine hohle, flexible Komponente, wie z. B. ein Hohlrohr, einschiebbar ist, um die flexible Komponente zu versteifen. Die Sondeneinheit hat eine flexible Sondenhülle und einen inneren Sondendraht, wobei das distale Ende des letzteren in bestimmter Weise vorgekrümmt ist. Die Sondeneinheit dient zum Versteifen beispielsweise eines Elektrodenkabels während des Vorschiebens des Kabels durch eine Vene zum Herzen eines Patienten.

Aus der NL-A-9 500 283 ist ein Katheter bekannt, welcher an seinem vorderen Ende zwei hintereinander angeordnete Ballone zum Dilatieren von Gefässen aufweist. Die Ballone sind über Anschlüsse jeweils mit einem unter Druck stehenden Medium beaufschlagbar.

Eine Führungsdraht genannte Führungseinrichtung ist beispielsweise aus der DE 100 17 147 A1 bekannt. Dieser Führungsdraht besteht aus einem äusseren Drahtstrang, der als Hohlstrang ausgebildet ist, sowie aus einem darin verschiebbaren Innenstrang. Der Drahtstrang hat an seinem distalen Ende eine gebogene Führungsspitze, die bei der Verlegung des Führungsdrahtes als Steuerungselement oder Wegfinder wirkt.

Die genannten Führungseinrichtungen bzw. Führungsdrähte werden in einen Körpergang, beispielsweise in ein Gefäss, wie eine Vene oder Arterie, eingeführt, damit nachfolgend entlang dem Führungsdraht ein Katheter nachgeschoben werden kann. Mit dem Führungsdraht ist dabei in verschiedene, enge Abzweigungen einzudringen, wobei nachträglich dann der relativ steife Katheter nachzuführen ist. Bei dem letztgenannten Führungsdraht ist die Krümmung am distalen Ende vorgegeben, so dass der entsprechend vorgeformte Führungskatheter mit der richtigen Krümmung auszuwählen ist. Der Durchmesser eines solchen Führungskatheters ist relativ gross, da man ihn als äussere Führung verwendet. In einem zweiten Schritt ist nämlich durch den Hohlraum des Führungskatheters der eigentliche Therapiekatheter, zum Beispiel ein Ballonkatheter, nachzuschieben. Der äussere Führungskatheter ist im vorderen Bereich aufgrund seiner Krümmung relativ dick. Ausserdem besteht die Gefahr, dass der vordere Teil des Führungsdrahtes bei einer Verzweigung des Gefässes beim Nachführen beispielsweise eines relativ starren Therapie-Katheters ohne Verwendung eines Führungskatheters wieder aus dem Zielgefäss herausrutscht.

Eine Führungseinrichtung nach dem Oberbegriff des Patentanspruchs 1 ist aus der US-A-5 337 733 bekannt. Diese Führungseinrichtung umfasst zwischen einer inneren Wand und einer äusseren Wand einen dazwischen liegenden Raum, welcher nach aussen hin abdichtbar ist. Die äussere Wand wird durch einen flexiblen Schlauch gebildet. Die innere Wand wird ebenfalls durch einen Schlauch gebildet, dieser ist im Gegensatz zur äusseren Wand in radialer Richtung nach innen hin deformierbar, wenn ein Fluid in den Zwischenraum eingeleitet wird. Ausserdem kann sich die äussere Wand durch Evakuieren des Zwischenraumes gegen die innere Wand legen, um die Führungseinrichtung steif zu machen. Aussenwand und Innenwand können über Eingriffselemente miteinander in Eingriff stehen.

Eine Führungseinrichtung nach dem Obergriff des Patentanspruchs 1 ist auch aus der US-A-5 706 827 bekannt.

Der Erfindung liegt die Aufgabe zugrunde, eine Führungseinrichtung der eingangs erwähnten Art zu schaffen, welche einfacher handhabbar, vor allem auf der einen Seite ausreichend flexibel, auf der anderen Seite aber auch hinreichend steif ist.

Diese Aufgabe wird erfindungsgemäss durch eine Führungseinrichtung mit den Merkmalen des Patentanspruchs 1 gelöst. Vorteilhafte Weiterbildungen sind Gegenstand der abhängigen Ansprüche.

Erfindungsgemäss ist die Steuereinrichtung derart ausgebildet, dass magnetische Felder unterschiedlicher Polarität entlang dem ersten Drahtstrang und entlang dem zweiten Drahtstrang zum wahlweisen Herbeiführen einer gegenseitigen Anziehung der Stränge erzeugbar sind. Dies hat den Vorteil, dass die einzelnen Stränge und damit der Führungseinrichtung insgesamt äusserst flexibel sind, so lange kein magnetisches Feld erzeugt wird, dass die Führungseinrichtung hingegen durch die gegenseitige Anziehung der Stränge hinreichend steif ist, sofern die magnetischen Felder erzeugt werden. Damit ist die erfindungsgemässe Führungseinrichtung auf der einen Seite, beispielsweise beim Einführen der Einrichtung in einen Körpergang, ausreichend flexibel, beim Nachführen des Katheters insbesondere im Bereich einer Abzweigung des Gefässes hingegen hinreichend steif, so dass die Gefahr eines Herausrutschens der Führungseinrichtung aus dem Zielgefäss weitgehend ausgeschlossen ist. Eine solche Führungseinrichtung verbindet damit zwei eher gegensätzliche Eigenschaften, nämlich einmal eine ausreichende Flexibilität und zum anderen eine hinreichende Steifigkeit, je nach dem, welche Eigenschaft bei der Handhabung der Führungseinrichtung gerade gewünscht ist.

Vorteilhafterweise sind der erste Drahtstrang und/oder der zweite Drahtstrang aus einem magnetisierbaren Material, insbesondere aus einem weichmagnetischen Werkstoff, gefertigt. Dadurch ist es möglich, die magnetischen Felder, wie gewünscht, aufbauen und gegebenenfalls auch wieder abbauen zu können, je nach dem, welche Flexibilität bzw. Steifigkeit für die Führungseinrichtung im praktischen Anwendungsfall gerade erforderlich ist. Gemäss einer anderen Weiterbildung der Erfindung sind der erste Drahtstrang und/oder der zweite Drahtstrang aus einem nichtmagnetisierbaren Material gefertigt und mit einer magnetisierbaren Beschichtung versehen. Dadurch kann jeder einzelne Drahtstrang aus einer Vielzahl von auszuwählenden Werkstoffen gefertigt sein. Es ist demnach nicht erforderlich, den gesamten Drahtstrang aus dem magnetisierbaren Material zu fertigen; es kann vielmehr ausreichen, lediglich den äusseren Mantel des Drahtstranges mit der magnetisierbaren Beschichtung zu versehen, damit ein solcher Drahtstrang ebenfalls magnetisierbar ist.

Gemäss einer vorteilhaften Weiterbildung der Erfindung sind der erste Drahtstrang und/oder der zweite Drahtstrang als Massivkörper oder als Hohlkörper ausgebildet, wobei vorzugsweise in dem Fall, dass beide Drahtstränge als Hohlkörper geformt sind, die Steuereinrichtung eine sich in jedem Drahtstrang befindende magnetisierbare Flüssigkeit aufweist. In dem letztgenannten Fall kann der Hohlkörper jedes Drahtstranges vollständig aus nichtmagnetisierbarem Material bestehen, da die gegenseitige Anziehung der Drahtstränge durch die in jedem Hohlkörper sich befindende magnetisierbare Flüssigkeit herbeigeführt werden kann.

Vorteilhafterweise sind die vorbeschriebenen magnetischen Felder durch elektrische Ströme, das heisst durch Anlegen einer elektrischen Spannung an die Drahtstränge, erzeugbar. Die magnetischen Felder können dann aufgrund elektrischer Felder durch einfaches Einschalten bzw. Ausschalten der elektrischen Spannung erzeugt werden. Die magnetischen Felder können elektromagnetisch oder einfach, wie zuvor erwähnt, mit Hilfe eines elektrischen Stromes, welcher durch einen Leiter fliesst, erzeugt werden. Die elektrischen und damit auch die magnetischen Felder können daher äusserst schnell auf- und abgebaut werden. Eine elektrische Spannung kann auf einfache Weise und ohne einen Eingriff in den zu untersuchenden Körper unmittelbar an die Führungseinrichtung angelegt werden, so dass negative Auswirkungen auf den zu untersuchenden menschlichen oder tierischen Körper weitgehend ausgeschlossen sind.

Gemäss einer Weiterbildung der Erfindung sind die Drahtstränge nebeneinander und parallel zueinander, vorzugsweise zueinander verdrillt, angeordnet. Bei einer besonders platzsparenden Ausführungsform sind die Drahtstränge konzentrisch zueinander angeordnet, wobei vorzugsweise einer der Drahtstränge zentral innenliegend und der andere der Drahtstränge wendelförmig, radial aussenliegend, um ersteren herum angeordnet ist. Ein wendelförmiger, äusserer Drahtstrang trägt mit zu einer besonders guten Flexibilität der Führungseinrichtung bei, so dass diese beispielsweise mit einem relativ engen Radius gekrümmt werden kann.

Gemäss einer bevorzugten Weiterbildung der Erfindung ist der erste Drahtstrang zentral innenliegend angeordnet und sind um den Aussenumfang des ersten Drahtstranges herum mehrere zweite Drahtstränge vorzugsweise gleichmässig voneinander beabstandet angeordnet. Damit ist auf der einen Seite eine gute Flexibilität der Führungseinrichtung, auf der anderen Seite eine ausreichende Steifigkeit der Führungseinrichtung dann gegeben, wenn sich der erste Drahtstrang und die zweiten Drahtstränge gegenseitig anziehen.

Vorteilhafterweise sind die Drahtstränge derart ausgebildet, dass sie bei Erzeugung eines magnetischen/elektrischen Feldes zum Herbeiführen einer gegenseitigen Anziehung flächig aneinander liegen. Damit ist ein guter Kontakt zwischen den Drahtsträngen ermöglicht und eine Relativbewegung zwischen den Drahtsträngen bei in Kraft gesetzten Feldern weitgehend verhindert, wodurch die Steifigkeit der gesamten Führungseinrichtung in der aktuellen, im Körper befindlichen Krümmung verbessert ist.

Gemäss einer anderen Weiterbildung der Erfindung sind die magnetischen Felder dauermagnetisch erzeugbar, wobei vorzugsweise jeder Drahtstrang entlang seiner Länge und in radialer Richtung abwechselnd umgekehrt polarisiert ist. Diese Ausführungsform hat den Vorteil, dass eine gegenseitige Anziehung bzw. Abstossung der einzelnen Drahtstränge durch eine einfache Verschiebung der Drahtstränge zueinander in axialer Richtung realisierbar ist. Da sich die Polarisierung längs jedes Drahtstrangs fortlaufend ändert, kann eine geringe axiale Verschiebung der Drahtstränge zueinander bereits die gewünschte Abstossung oder Anziehung bewirken.

Vorteilhafterweise ermöglicht die Steuereinrichtung einen gegenseitigen Kontakt oder Eingriff zueinander weisender Grundflächen der Drahtstränge vorzugsweise in Form einer Verzahnung und durch Einleiten eines unter Druck stehenden Fluids, vorzugsweise einer Flüssigkeit oder eines Gases, in die Spalte zwischen den Drahtsträngen ein Trennen der Grundflächen voneinander. Durch einen gegenseitigen Kontakt oder Eingriff der zueinander weisenden Flächen der Drahtstränge ist es möglich, die Reibung zwischen den genannten Flächen stark zu erhöhen und damit eine Relativbewegung zwischen den genannten Drahtsträngen weitgehend zu unterbinden. Auf der anderen Seite kann der Führungseinrichtung äusserst flexibel sein, wenn die Flächen durch die vorgenannte Massnahme voneinander getrennt worden sind.

Gemäss einer bevorzugten Weiterbildung der Erfindung ist der gegenseitige Kontakt oder Eingriff der zueinander weisenden Flächen der Drahtstränge durch Abziehen des Fluids vorzugsweise unter zusätzlichem Anlegen eines Vakuums herbeiführbar. Damit ist sichergestellt, dass die einzelnen Drahtstränge auch nach häufigem Gebrauch der Führungseinrichtung eng aneinander anliegen und somit dazu beitragen können, eine Relativbewegung zwischen den Drahtsträngen zu verhindern. Gegebenenfalls ist diese Wirkung schon durch das Anlegen eines Vakuums zum Erreichen eines gegenseitigen Kontaktes der zueinander weisenden Flächen der Drahtstränge erzielbar, ohne dass es an den zueinander weisenden Grundflächen einer bestimmten Bearbeitung oder des Vorsehens einer Verzahnung bedarf.

Ausführungsbeispiele des Erfindungsgegenstandes werden nachfolgend anhand der Zeichnung näher erläutert, wobei alle beschriebenen und/oder bildlich dargestellten Merkmale für sich oder in beliebiger Kombination den Gegenstand der vorliegenden Erfindung unabhängig von ihrer Zusammenfassung in den Ansprüchen oder deren Rückbeziehung bilden. Es zeigen:
- Fig. 1: einen schematischen, stark vergrösserten Schnitt durch eine Führungseinrichtung, insbesondere zum Positionieren von Kathetern in einem Körpergang, gemäss einer nicht zum Umfang der Erfindung gehörenden Ausführungsform;
- Fig. 2: einen schematischen, stark vergrösserten Schnitt durch eine Führungseinrichtung, insbesondere zum Positionieren von Kathetern in einem Körpergang, gemäss einer erfindungsgemässen Ausführungsform;
- Fig. 3: einen schematischen, stark vergrösserten Schnitt durch eine Führungseinrichtung gemäss einer weiteren erfindungsgemässen Ausführungsform;
- Fig. 4: einen schematischen, stark vergrösserten Schnitt durch eine Führungseinrichtung gemäss einer anderen erfindungsgemässen Ausführungsform;
- Fig. 5: eine schematische Draufsicht auf die Führungseinrichtung gemäss Fig. 4, in welcher die beiden Drahtstränge voneinander beabstandet dargestellt sind;
- Fig. 6: eine schematische Draufsicht auf die in Fig. 4 gezeigte Führungseinrichtung, in welcher die beiden Drahtstränge voneinander beabstandet und gegeneinander verschoben dargestellt sind;
- Fig. 7: einen schematischen Schnitt durch eine Führungseinrichtung gemäss einer weiteren Ausführungsform;
- Fig. 8: einen schematischen Schnitt durch eine Führungseinrichtung gemäss einer anderen Ausführungsform; und
- Fig. 9: einen schematischen Schnitt durch eine Führungseinrichtung gemäss einer weiteren Ausführungsform.

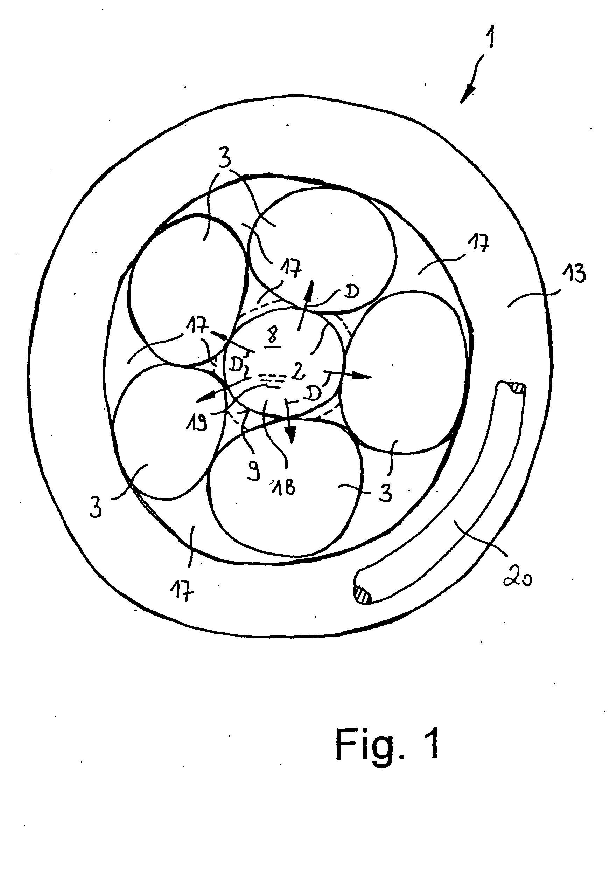

Zunächst wird darauf hingewiesen, dass in Fig. 1 der besseren Übersicht halber die einen Schnitt symbolisierenden Schraffuren weggelassen sind.

In Fig. 1 ist eine Führungseinrichtung 1 in einem schematischen Querschnitt gezeigt. Die Führungseinrichtung 1 ist gemäss einer nicht zum Umfang der Erfindung gehörenden Ausführungsform ausgestaltet. Die Führungseinrichtung 1 hat gemäss dieser Ausführungsform einen ersten Strang 2 in Form eines zentralen Schlauches oder Ballons. Dieser ist aus einem dehnbaren, elastischen Material gefertigt und auf seiner Umfangsfläche 9 von mehreren zweiten Strängen 3 aus Draht unter Belassung zwickelartiger Zwischenräume 17 dicht umgeben. In der in Fig. 1 gezeigten Ausführungsform sind fünf zweite Stränge 3 um di äusseren Umfangsfläche 9 des ersten Stranges 2 herum angeordnet. Die zweiten Stränge 3 sind gemäss der gezeigten Ausführungsform eng von einer Aussenhülle 13 umgeben, welche vorzugsweise aus einem nicht oder wenig dehnbaren Material gefertigt ist.

Es ist klar, dass die in Fig. 1 gezeigte Ansicht eine sehr starke Vergrösserung der erfindungsgemässen Führungseinrichtung 1 darstellt. Es ist ferner klar, dass auch weniger oder mehr als fünf zweite Stränge 3, welche in Form jeweils eines Drahtstranges ausgebildet sind, vorgesehen sein können.

Den Strängen 2, 3 ist eine Einrichtung 8 zugeordnet, mittels der eine Relativbewegung zwischen den Strängen gestattet oder weitgehend verhindert werden kann. Die Steuereinrichtung 8 ist derart ausgebildet, dass der erste Strang 2 in seinem in Fig. 1 in gestrichelten Linien angedeuteten, gedehnten Zustand auf die mehreren zweiten Stränge 3 einen gemäss Fig. 1 radial wirkenden Druck gemäss der Pfeile D ausübt. Dazu ist der Innenraum 18 des ersten Stranges 2, nämlich des Schlauchs oder Ballons, mittels der Steuereinrichtung 8 mit einem unter Druck stehenden Fluid 19, vorzugsweise einer Flüssigkeit, beaufschlagbar. Das Fluid 19 ist in Fig. 1 lediglich schematisch angedeutet. Es ist klar, dass das Fluid den Innenraum 18 des Stranges 2 üblicherweise vollständig ausfüllt.

Wie in Fig. 1 angedeutet, berühren sich erster Strang 2 und die mehreren zweiten Stränge 3 sowie die zweiten Stränge 3 untereinander schon im nicht gedehnten Zustand des ersten Stranges 2 gegenseitig. In dem in Fig. 1 gestrichelt angedeuteten, gedehnten Zustand des ersten Stranges 2 liegen erster Strang und die mehreren zweiten Stränge sowie die zweiten Stränge untereinander vorzugsweise flächig aneinander.

Ferner ist in Fig. 1 angedeutet, dass die Aussenhülle 13 einen in Längsrichtung gewundenen Spiraldraht 20 aufweist, von dem in Fig. 1 lediglich ein Teilstück gezeigt ist.

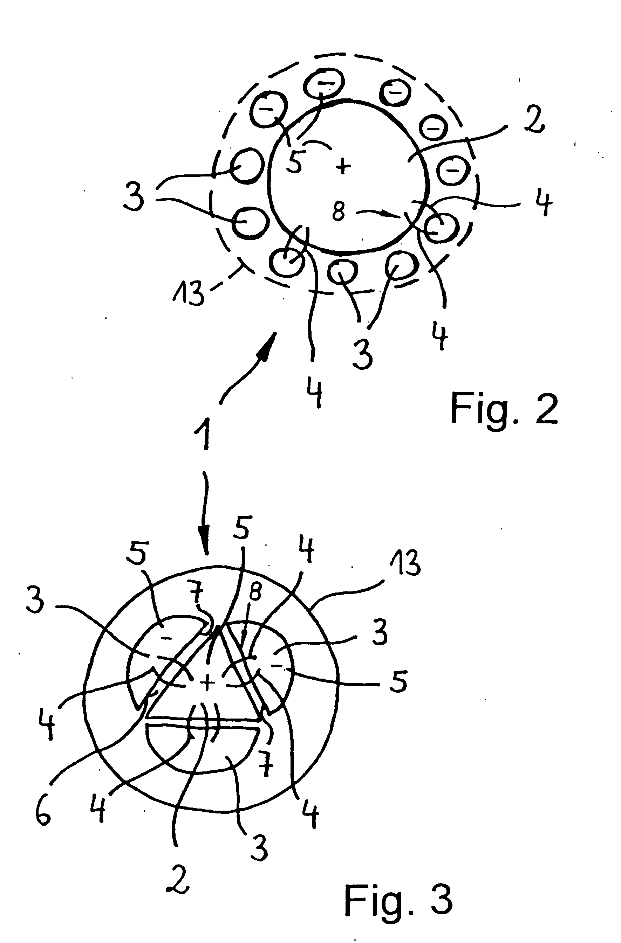

Ferner wird darauf hingewiesen, dass auch in den Fig. 2 bis 4 sowie Fig. 7 der besseren Übersicht halber die einen Schnitt symbolisierenden Schraffuren weggelassen worden sind und dass der Begriff "Draht" hier in einem allgemeinen, umfassenden Sinn für einen dünnen, länglichen Körper jedweden Materials verwendet wird.

In den Fig. 2 bis 4 sowie 7 bis 9 sind schematisch jeweils Schnitte durch verschiedene Ausführungsformen einer erfindungsgemässen Führungseinrichtung 1 insbesondere zum Positionieren von nicht näher gezeigten Kathetern in einem nicht dargestellten Körpergang dargestellt. Die Führungseinrichtung ist hier jeweils in Form eines sogenannten Führungsdrahtes ausgebildet. Die Katheter sind beispielsweise Röhrchen aus Metall, Glas, Kunststoff oder Gummi zur Einführung in Körperorgane, wie zum Beispiel in die Harnblase, um diese zu entleeren, zu füllen, zu spülen oder zu untersuchen. Der Körpergang ist beispielsweise ein Gefäss eines menschlichen oder tierischen Körpers, wie zum Beispiel eine Vene oder eine Arterie.

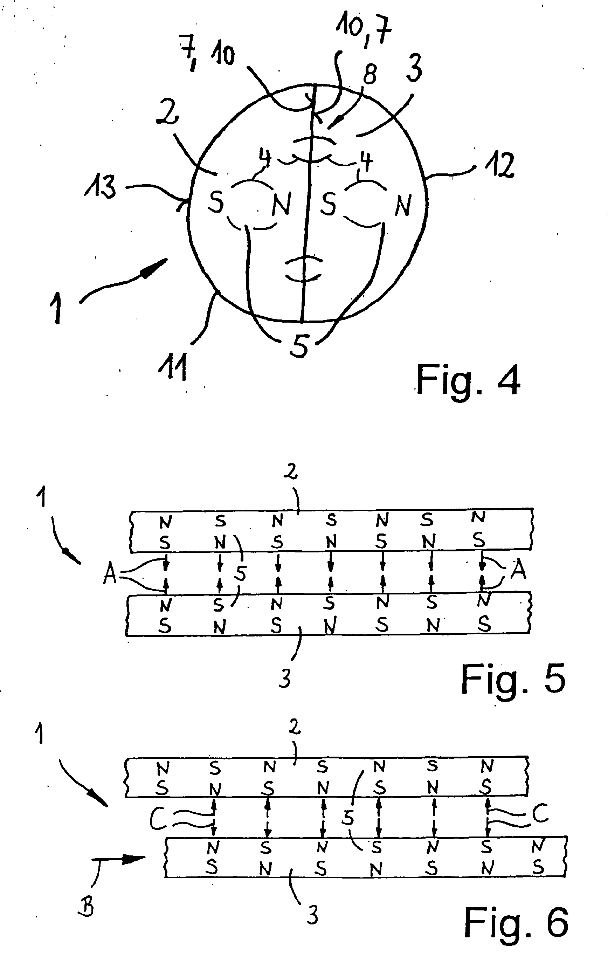

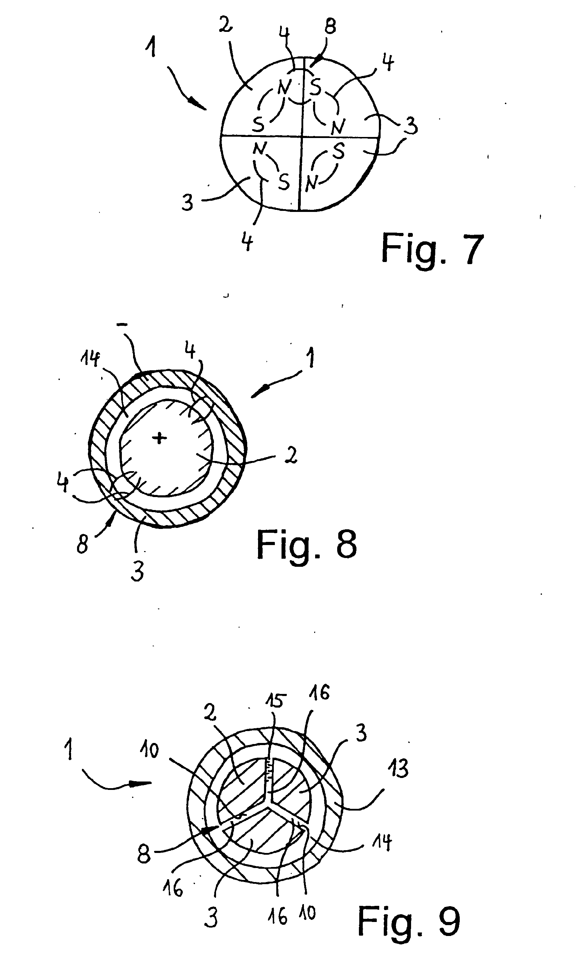

Die Stränge 2, 3 werden nachfolgend Drahtstränge bezeichnet. Die Führungseinrichtung 1 hat einen langgestreckten, ersten Drahtstrang 2 und wenigstens einen langgestreckten, zweiten Drahtstrang 3, welcher nahe dem ersten Drahtstrang 2 verläuft. In dem Ausführungsbeispiel gemäss Fig. 2 sind 11 zweite Drahtstränge 3, in dem Ausführungsbeispiel gemäss Fig. 3 drei zweite Drahtstränge 3, in dem Ausführungsbeispiel gemäss Fig. 4 ein zweiter Drahtstrang 3, in dem Ausführungsbeispiel gemäss Fig. 7 drei zweite Drahtstränge 3, in dem Ausführungsbeispiel gemäss Fig. 8 ein zweiter Drahtstrang 3 und in dem Ausführungsbeispiel gemäss Fig. 9 zwei zweite Drahtstränge 3 vorgesehen.

Erfindungsgemäss ist den Drahtsträngen 2, 3 die Einrichtung 8 zugeordnet, mittels der die Möglichkeit, eine Relativbewegung zwischen den Drahtsträngen 2, 3 zuzulassen oder zumindest zu erschweren, gezielt steuerbar ist. Diese Einrichtung 8 wird nachfolgend Steuereinrichtung genannt.

Die Steuereinrichtung 8 ist erfindungsgemäss derart ausgebildet, dass magnetische Felder 4 unterschiedlicher Polarität 5 entlang dem ersten Drahtstrang 2 auf der einen Seite und entlang dem zweiten Drahtstrang 3 auf der anderen Seite zum wahlweisen Herbeiführen einer gegenseitigen Anziehung der Drahtstränge 2, 3 erzeugbar sind. Die auf- bzw. abbaubaren Felder 4 oder Kräfte sind in den Fig. 2 bis 8 lediglich schematisch angedeutet, wobei klar ist, dass derartige Felder zwischen jedem ersten Drahtstrang 2 und jedem zweiten Drahtstrang 3 bestehen, sofern diese Felder erzeugt worden sind. Die unterschiedliche Polarität 5 von erstem Drahtstrang 2 und zweitem Drahtstrang 3 ist beispielsweise in den Fig. 2, 3 sowie 8 durch die Bezeichnung "+" oder "-" und in den Fig. 4 bis 7 durch die Buchstaben "N" und "S" angedeutet, wobei "+" und "-" für eine positive bzw. negative elektrische Ladung und "N" für Nordpol und "S" für Südpol des Magnetfeldes stehen.

Gemäss einer Ausführungsform sind der erste Drahtstrang und der zweite Drahtstrang bzw. der erste Drahtstrang oder der zweite Drahtstrang aus einem magnetisierbaren Material, insbesondere aus einem weichmagnetischen Werkstoff, gefertigt. Gemäss einer anderen Ausführungsform sind der erste Drahtstrang und der zweite Drahtstrang bzw. der erste Drahtstrang oder der zweite Drahtstrang aus einem nichtmagnetisierbaren Material gefertigt und auf ihrer Oberfläche mit einer magnetisierbaren Beschichtung 7 versehen, wobei es möglich ist, die Beschichtung nur in den Bereichen der Drahtstränge 2, 3 vorzusehen, die zum jeweils anderen Drahtstrang gerichtet sind. Dies sind bei der Ausführungsform gemäss Fig. 3 beispielsweise die Grundflächen 6 der teilzylinderförmigen zweiten Drahtstränge 3. In dem Ausführungsbeispiel gemäss Fig. 4 kann die magnetisierbare Beschichtung 7 auf den Grundflächen 10 der Teilzylinderhälften 11 und 12 vorgesehen sein. Gemäss den dargestellten Ausführungsbeispielen sind der erste Drahtstrang 2 und/oder der zweite Drahtstrang 3 als Massivkörper (siehe Fig. 2 bis 4, 7) oder als Hohlkörper (siehe Fig. 8 und 9 in Bezug auf den zweiten Drahtstrang 3) ausgebildet.

Gemäss einer nicht dargestellten Ausführungsform der Erfindung kann in dem Fall, dass beide Drahtstränge als Hohlkörper geformt sind, sich in jedem Drahtstrang eine magnetisierbare Flüssigkeit befinden, so dass beim Anlegen magnetischer Felder eine gegenseitige Anziehung der nebeneinander oder konzentrisch ineinander angeordneten Drahtstränge möglich ist. Eine magnetisierbare Flüssigkeit ist beispielsweise eine kolloidale, besonders stabilisierte Suspension magnetischer oder magnetisierbarer Partikel. Meist werden Teilchen von etwa 10 nm verwendet, die durch Umhüllung mit einer oberflächenaktiven Substanz, wie zum Beispiel Ölsäure, daran gehindert sind, sich unter der Wirkung der magnetischen Wechselwirkungen zusammen zu lagern. Als Trägerflüssigkeit können Wasser, aber auch Öle und verschiedene andere Lösungsmittel dienen. Eine magnetisierbare Flüssigkeit lässt sich durch ein Magnetfeld in jeder Lage festhalten. Dieser technische Effekt lässt sich auch zum Versteifen einer Führungseinrichtung nutzen.

Die magnetischen Felder 4 sind durch Anlegen einer nicht gezeigten elektrischen Spannung an die Drahtstränge 2, 3 bzw. die magnetisierbare Beschichtung 7 erzeugbar.

Die Drahtstränge 2, 3 sind gemäss den Fig. 2 bis 7 nebeneinander und parallel zueinander angeordnet. In Fig. 7 ist ein schematischer Schnitt durch eine weitere Ausführungsform einer Führungseinrichtung 1 gezeigt, bei welchem die Ausführungsform gemäss Fig. 4 nochmals halbiert ist, so dass sich auch bei dieser Ausführungsform die einzelnen Drahtstränge 2, 3 nebeneinander und parallel zueinander befinden.

Gemäss einer nicht näher gezeigten Ausführungsform ist es auch möglich, die Drahtstränge 2, 3 zueinander zu verdrillen.

Gemäss der in Fig. 8 gezeigten Ausführungsform sind die Drahtstränge 2, 3 konzentrisch zueinander angeordnet, wobei sich der erste Drahtstrang 2 mittig innerhalb des als Hohlkörper ausgebildeten, zweiten Drahtstranges 3 befindet.

Gemäss einer weiteren, nicht näher gezeigten Ausführungsform der Erfindung ist einer der Drahtstränge, beispielsweise der erste Drahtstrang 2, zentral innenliegend und der andere der Drahtstränge, beispielsweise der zweite Drahtstrang 3, wendelförmig, radial aussen liegend, um ersteren herum angeordnet.

Wie in der in Fig. 2 näher gezeigten Ausführungsform angedeutet, ist der erste Drahtstrang 2 zentral innenliegend angeordnet und mit einem kreisförmigen Querschnitt versehen. Um den Aussenumfang des ersten Drahtstranges 2 herum sind mehrere zweite Drahtstränge 3, im gewählten Ausführungsbeispiel gleichmässig voneinander beabstandet angeordnet. Beispielsweise sind im Falle der Ausführungsform der Fig. 2 elf zweite Drahtstränge 3 vorgesehen, die jeweils ebenfalls einen kreisförmigen Querschnitt aufweisen, wobei der Durchmesser des ersten Drahtstranges 2 deutlich grösser als derjenige der zweiten Drahtstränge 3 gewählt ist und der Abstand der jeweiligen zweiten Drahtstränge 3 vom ersten Drahtstrang 2 deutlich geringer als zum benachbarten zweiten Drahtstrang 3 ist.

Bei der in Fig. 3 gezeigten Ausführungsform hat der erste Drahtstrang 2 einen Querschnitt in Form eines gleichseitigen Dreiecks, während die Grundfläche 6 jedes der zweiten Drahtstränge 3 in diesem Ausführungsbeispiel derart ausgebildet ist, dass die Breite der Grundfläche 6 etwa der Länge einer der Dreieckseiten des Querschnitts des ersten Drahtstranges in Form eines gleichseitigen Dreiecks entspricht. Die zweiten Drahtstränge 3 sind teilzylindrisch oder zylindersegmentartig ausgebildet. Die Drahtstränge 2 und 3 sind gemäss den Ausführungsformen der Fig. 2 und 3 ferner von einer Aussenhülle 13 umgeben, die in Fig. 2 lediglich gestrichelt angedeutet ist.

Bei der in Fig. 4 dargestellten Ausführungsform sind erster Drahtstrang 2 und zweiter Drahtstrang 3 etwa identisch zueinander ausgebildet und halbzylindrisch geformt. Die beiden Teilzylinderhälften 11 und 12 ergeben damit im zusammengesetzten bzw. - gelegten Zustand einen kreisförmigen Querschnitt. Auch in diesem Fall sind die einzelnen Drahtstränge 2, 3 vorzugsweise von einer Aussenhülle 13 umgeben.

Gemäss den in den Fig. 4 bis 7 dargestellten Ausführungsformen der Erfindung sind die magnetischen Felder 4 sowohl innerhalb jedes Drahtstrangs als auch von einem Drahtstrang zum andern dauermagnetisch erzeugbar, wobei, wie im einzelnen in einer Draufsicht auf die Führungseinrichtung 1 gemäss Fig. 4 in Fig. 5 gezeigt, in der die einzelnen Drahtstränge allerdings voneinander getrennt sind, jeder Drahtstrang 2, 3 entlang seiner Länge und in radialer Richtung abwechselnd umgekehrt polarisiert ist. Dadurch ergibt sich bei einer axialen Anordnung der Drahtstränge 2, 3 gemäss Fig. 5 eine gegenseitige Anziehung der Drahtstränge 2, 3, wie dies durch die zueinander gerichteten Pfeile A in Fig. 5 verdeutlicht ist.

Wird nun, wie in Fig. 6 angedeutet, der zweite Drahtstrang 3 in Richtung des Pfeils B relativ zu dem ersten Drahtstrang 2 in axialer Richtung verschoben, so stossen sich die Drahtstränge 2, 3 voneinander ab, da gleiche Polaritäten sich in den Drahtsträngen einander gegenüberliegen. Dies ist durch die voneinander weg gerichteten Pfeile C in Fig. 6 verdeutlicht. Es ist klar, dass der in Fig. 6 gezeigte Effekt des sich Abstossens der Drahtstränge ausgehend von der Anordnung gemäss Fig. 5 auch dadurch erreicht werden kann, dass der erste Drahtstrang 2 relativ zu dem zweiten Drahtstrang 3 in axialer Richtung verschoben wird.

Bei der in Fig. 7 gezeigten Ausführungsform sind die einzelnen Drahtstränge 2, 3 viertelzylinderförmig und etwa identisch ausgebildet, so dass vier zueinander gerichtete und aneinander anliegende Drahtstränge einen Kreisquerschnitt ergeben. Auch bei dieser Ausführungsform sind die einzelnen Polaritäten durch die Kurzbezeichnungen "N" und "S" mit den magnetischen Feldern 4 verdeutlicht, welche einmal innerhalb jedes Drahtstrangs, dann aber auch von einem Drahtstrang zum andern verlaufen.

Auch in diesem Fall könnten die einzelnen Drahtstränge 2, 3 analog zu der in den Fig. 5 und 6 verdeutlichten Ausführungsform in axialer Richtung gegeneinander verschoben werden, wodurch sich, wie in Fig. 6 angedeutet, in einander gegenüberliegenden Drahtsträngen gleiche Polaritäten ergeben können, so dass sich die Drahtstränge voneinander abstossen. Insofern sind die Drahtstränge 2, 3 in den Ausführungsformen der Fig. 4 bis 7 in Form von Dauermagneten gebildet.

Bei einem anderen Ausführungsbeispiel können die Drahtstränge 2, 3 jeweils auch einen kreisförmigen Querschnitt aufweisen und nebeneinander angeordnet sein (nicht gezeigt), wobei die zueinander weisenden Flächen dieser Drahtstränge auch derart ausgestaltet sein können, dass beim gegenseitigen Anziehen dieser Drahtstränge nicht eine linienförmige, sondern eine flächige Berührung der Drahtstränge möglich ist.

Es ist klar, dass die gegenseitigen Abstände sowohl der ersten Drahtstränge zu den zweiten Drahtsträngen als auch der zweiten Drahtstränge, sofern mehrere vorhanden sind, zueinander in den Fig. 2 bis 9 stark vergrössert dargestellt sind und dass insbesondere bei den Ausführungsbeispielen der Fig. 2 und 3 die Aussenhülle 13 auch eng an den zweiten Drahtsträngen 3 anliegen kann.

Bei der in Fig. 8 gezeigten Ausführungsform der Erfindung hat der erste Drahtstrang 2 einen kreisförmigen Querschnitt und befindet sich in dem zweiten Drahtstrang 3, welcher rohrförmig mit ringförmiger Querschnittsfläche ausgebildet ist. Auch bei dieser Ausführungsform ist klar, dass der zwischen dem ersten Drahtstrang 2 und dem zweiten Drahtstrang 3 gezeichnete Abstand stark vergrössert ist und dass in der Praxis der Aussendurchmesser des ersten Drahtstranges 2 nur geringfügig kleiner als der Innendurchmesser des zweiten Drahtstranges 3 ist. Sofern gewünscht, kann sich in dem ringförmigen Innenraum 14 zwischen erstem Drahtstrang 2 und zweitem Drahtstrang 3 auch eine Gleitflüssigkeit befinden. Es ist aber auch möglich, eine solche Gleitflüssigkeit wegzulassen.

Eine weitere Ausführungsform der Führungseinrichtung 1 ist in einem schematischen Querschnitt in Fig. 9 dargestellt. In der Aussenhülle 13 befinden sich drei Drahtstränge, nämlich ein erster Drahtstrang 2 und zwei zweite Drahtstränge 3, welche etwa identische Querschnitte haben. Die Steuereinrichtung 8 ist bei dieser Ausführungsform derart ausgebildet, dass sie einen gegenseitigen Kontakt oder Eingriff zueinander weisender Grundflächen 10 vorzugsweise in Form einer in Fig. 9 lediglich schematisch angedeuteten Verzahnung 15, beispielsweise in Form feiner auf den zueinanderweisenden Grundflächen der Drahtstränge 2, 3 befindlicher Häarchen, und durch Einleiten eines unter Druck stehenden Fluids, vorzugsweise einer Flüssigkeit oder eines Gases, in die Spalte 16 zwischen den Drahtstängen 2, 3 ein Trennen der Grundflächen 10 voneinander ermöglicht. Ebenso kann das Fluid in den ringförmigen Innenraum 14 zwischen den Drahtsträngen 2, 3 und der Aussenhülle 13 eingeleitet werden. Der gegenseitige Kontakt oder Eingriff der zueinander weisenden Grundflächen 10 der teilzylinderförmig ausgestalteten Drahtstränge 2, 3 ist beispielsweise durch Abziehen des Fluids vorzugsweise unter zusätzlichem Anlegen eines Vakuums herbeiführbar.

Im Fall der in den Fig. 3 bis 8 gezeigten Ausführungsformen sind die Drahtstränge 2, 3 derart ausgebildet, dass sie bei Erzeugung eines magnetischen Feldes 4 flächig aneinander anliegen und damit zu einer ausgeprägten Versteifungsmöglichkeit der Führungseinrichtung 1 beitragen können. Es ist klar, dass erster Drahtstrang 2 und zweiter Drahtstrang 3 positiv oder negativ geladen werden können, um die zum Herbeiführen einer gegenseitigen Anziehung der Drahtstränge erforderlichen Magnetfelder zu erzeugen. Durch die gegenseitige Anziehung der Drahtstränge wird die Versteifung der Führungseinrichtung 1 erreicht. Eine solche Versteifung ist in derjenigen Lage der Führungseinrichtung möglich, die der Draht in dem jeweiligen Körpergang gerade einnimmt.

Die Führungseinrichtung 1 bzw. der Führungsdraht hat die Fähigkeit, sich nicht ohne weiteres zu verdrehen; sie/er ist damit torsionsfest. Sie/er weist ferner eine Verkrümmungssteifigkeit, eine Schubfähigkeit und eine Nichtknickbarkeit auf. Sie/er ist ausreichend flexibel und gleitfähig und hat einen Durchmesser von beispielsweise 0,9 oder 1 mm. Damit ist die erfindungsgemässe Führungseinrichtung handhabbar, ohne ein in Bezug auf den Querschnitt der Führungseinrichtung grösseres Zusatzloch machen zu müssen. Ferner besteht eine weitgehende Flexibilität bezüglich der für die Stränge der Führungseinrichtung ausgewählten Materialien. Mit der erfindungsgemässen Führungseinrichtung können alle üblichen Katheter verwendet werden. Die Handhabung ist sicher und einfach.

Die erfindungsgemässe Führungseinrichtung ist, wie erwähnt, insbesondere zum Positionieren von Kathetern in einem Körpergang einsetzbar. Es ist aber auch möglich, eine solche Führungseinrichtung in der industriellen Technik einzusetzen, wo dann gegebenenfalls dickere Führungseinheiten Verwendung finden können.

Damit ist eine Führungseinrichtung geschaffen, die auf der einen Seite ausreichend flexibel, auf der anderen Seite aber auch hinreichend steif ist, so dass die erfindungsgemässe Führungseinrichtung leicht handhabbar ist.

## Patentansprüche

1. Führungseinrichtung insbesondere zum Positionieren von Kathetern in einem Körpergang, mit einem langgestreckten, ersten Strang (2), wenigstens einem langgestreckten, zweiten Strang (3), welcher nahe dem ersten Strang (2) verläuft, und mit einer den Strängen (2, 3) zugeordneten Einrichtung (8), mittels der die Möglichkeit, eine Relativbewegung zwischen den Strängen (2, 3) zuzulassen oder zumindest zu erschweren, gezielt steuerbar ist, **dadurch gekennzeichnet, dass** der erste Strang (2) und der zweite Strang (3) aus Draht gefertigt sind und die Steuereinrichtung (8) derart ausgebildet ist, dass magnetische Felder (4) unterschiedlicher Polarität (5) an dem ersten Drahtstrang (2) und an dem zweiten Drahtstrang (3) zum wahlweisen Herbeiführen einer gegenseitigen Anziehung der Drahtstränge (2, 3) erzeugbar sind.

2. Führungseinrichtung nach Anspruch 1, **dadurch gekennzeichnet, dass** der erste Strang (2) und/oder der zweite Strang (3) aus einem magnetisierbaren Material, insbesondere aus einem weichmagnetischen Werkstoff, gefertigt sind oder dass der erste Strang (2) und/oder der zweite Strang (3) aus einem nichtmagnetisierbaren Material gefertigt und mit einer magnetisierbaren Beschichtung (7) versehen sind.

3. Führungseinrichtung nach Anspruch 1 oder 2, **dadurch gekennzeichnet, dass** der erste Strang (2) und/oder der zweite Strang (3) als Massivkörper oder als Hohlkörper ausgebildet sind, wobei vorzugsweise in dem Fall, dass beide Stränge (2, 3) als Hohlkörper geformt sind; die Steuereinrichtung (8) eine sich in jedem Strang (2, 3) befindende magnetisierbare Flüssigkeit aufweist.

4. Führungseinrichtung nach einem der Ansprüche 1 bis 3, **dadurch gekennzeichnet, dass** die magnetischen Felder (4) durch Anlegen einer elektrischen Spannung an die Stränge (2, 3) aus Draht erzeugbar sind.

5. Führungseinrichtung nach einem der vorhergehenden Ansprüche, **dadurch gekennzeichnet, dass** die Stränge. (2, 3) aus Draht nebeneinander und parallel zueinander, vorzugsweise zueinander verdrillt, angeordnet sind oder dass die Stränge (2, 3) konzentrisch zueinander angeordnet sind, wobei vorzugsweise einer der Stränge (2, 3) zentral innenliegend und der andere der Stränge (3, 2) wendelförmig, radial aussenliegend, um ersteren herum angeordnet ist.

6. Führungseinrichtung nach Anspruch 5, **dadurch gekennzeichnet, dass** der erste Strang (2) zentral innenliegend angeordnet ist und um den Aussenumfang des ersten Stranges (2) herum mehrere zweite Stränge (3) vorzugsweise gleichmässig voneinander beabstandet angeordnet sind.

7. Führungseinrichtung nach einem der vorhergehenden Ansprüche, **dadurch gekennzeichnet, dass** die Stränge (2, 3) derart ausgebildet sind, dass sie bei Erzeugung eines magnetischen Feldes zum Herbeiführen einer gegenseitigen Anziehung flächig aneinander liegen.

8. Führungseinrichtung nach einem der vorhergehenden Ansprüche, **dadurch gekennzeichnet, dass** die magnetischen Felder (4) dauermagnetisch erzeugbar sind, wobei vorzugsweise jeder Strang (2, 3) aus Draht entlang seiner Länge und in radialer Richtung abwechselnd umgekehrt polarisiert ist.

9. Führungseinrichtung nach einem der vorhergehenden Ansprüche, **dadurch gekennzeichnet, dass** die Steuereinrichtung (8) einen gegenseitigen Kontakt oder Eingriff zueinander weisender Grundflächen (6, 10) der Stränge (2, 3), vorzugsweise in Form einer Verzahnung (15), und durch Einleiten eines unter Druck stehenden Fluids, vorzugsweise einer Flüssigkeit oder eines Gases, in die Spalte (16) zwischen den Strängen (2, 3) ein Trennen der Grundflächen (6, 10) voneinander ermöglicht.

10. Führungseinrichtung nach Anspruch 9, **dadurch gekennzeichnet, dass** der gegenseitige Kontakt oder Eingriff der zueinander weisenden Grundflächen (6, 10) der Stränge (2, 3) durch Abziehen des Fluids vorzugsweise unter zusätzlichem Anlegen eines Vakuums herbeiführbar ist.

## Claims

1. Guide device in particular for the positioning of catheters in a body duct with a long first thread (2), at least one long second thread (3), which runs close to the first thread (2) and a device (8) connected to the threads (2, 3) with which the possibility to permit relative movement between the wire threads (2, 3) or at least to make it more difficult can be controlled purposefully, **characterized by** the fact that the first thread (2) and the second thread (3) are made of wire and the control device (8) is finished in such a way that magnetic fields (4) of different polarities (5) can be generated along the first wire thread (2) and along the second wire thread (3) to bring about a mutual attraction of the wire threads (2, 3) at will.

2. Guide device in accordance with Claim 1, **characterized by** the fact that the first thread (2) and/or the second thread (3) is manufactured from a magetizable material, especially a weakly magnetizable material or that the first thread (2) and/or the second thread (3) is manufactured from a non-magnetizable material and is provided with a magnetizable coating (7).

3. Guide device in accordance with Claim 1 or 2, **characterized by** the fact that the first thread (2) and/or the second thread (3) is shaped as a solid body or as a hollow body, whereby in the case that both threads (2, 3) are shaped as hollow bodies, the control device (8) preferably exhibits a magnetizable fluid found in each wire thread (2, 3).

4. Guide device in accordance with one of the Claims 1 through 3, **characterized by** the fact that the magnetic fields (4) can be generated through the application of electric voltage to the threads (2, 3) made of wire.

5. Guide device in accordance with one of the previous Claims, **characterized by** the fact that the threads (2, 3) made of wire are arranged beside each other and parallel to each other, preferably twisted around each other, or that the wire threads (2, 3) are arranged concentric to each other, whereby preferably one of the threads (2, 3) is arranged centrally on the inside and the other of the threads (3, 2) is arranged spirally, radially to the outside around the first wire thread.

6. Guide device in accordance with Claim 5, **characterized by** the fact that the first thread (2) is arranged centrally on the inside and several second threads (3) are arranged around the outer circumference of the first thread (2), preferably uniformly spaced from each other.

7. Guide device in accordance with one of the previous Claims, **characterized by** the fact that the threads (2, 3) are finished in such a way that they lie flat against each other with the generation of a magnetic field to bring about mutual attraction.

8. Guide device in accordance with one of the previous Claims, **characterized by** the fact that the magnetic fields (4) can be generated permanently magnetically, whereby preferably each thread (2, 3) made of wire is polarized along its length and alternately oppositely in the radial direction.

9. Guide device in accordance with one of the previous Claims, **characterized by** the fact that the control device (8) enables mutual contact or attachment to each other of the basic surfaces (6, 10) of the threads (2, 3) facing each other, preferably in the form of teeth (15) and enables the separation of the basic surfaces (6, 10) from each other through the introduction of a fluid under pressure, preferably a liquid or a gas in the gap (16) between the threads (2, 3).

10. Guide wire in accordance with Claim 9, **characterized by** the fact that the mutual contact or attachment of the basic surfaces (6, 10) of the threads (2, 3) facing each other can be brought about through the removal of the fluid, preferably with the additional application of a vacuum.

## Revendications

1. Dispositif de guidage, en particulier pour le positionnement de cathéters dans un conduit corporel, comportant un premier fil (2) de grande longueur, au moins un deuxième fil (3) de grande longueur, qui court près du premier fil (2), et un dispositif (8), affecté à l'un des fils (2, 3), à l'aide duquel il est possible de commander d'une manière ciblée la possibilité d'autoriser ou d'au moins rendre difficile un déplacement relatif entre les fils (2, 3), **caractérisé en ce que** le premier fil (2) et le deuxième fil (3) sont fabriqués en un fil métallique, et le dispositif de commande (8) est configuré de telle sorte que des champs magnétiques (4), ayant des polarités (5) différentes, puissent être produits sur le premier fil métallique (2) et sur le deuxième fil métallique (3), pour provoquer à volonté une attraction réciproque des fils métalliques (2, 3).

2. Dispositif de guidage selon la revendication 1, **caractérisé en ce que** le premier fil (2) et/ou le deuxième fil (3) sont fabriqués en un matériau aimantable, en particulier en un matériau magnétique doux, ou **en ce que** le premier fil (2) et/ou le deuxième fil (3) sont fabriqués en un matériau non aimantable, et sont pourvus d'un revêtement aimantable (7).

3. Dispositif de guidage selon la revendication 1 ou 2, **caractérisé en ce que** le premier (2) et/ou le deuxième fil (3) sont réalisés sous forme d'un objet massif ou d'un corps creux, le dispositif de commande (8), de préférence dans le cas dans lequel les deux fils (2, 3) sont réalisés sous forme de corps creux, comportant un liquide aimantable, se trouvant dans chaque fil (2, 3).

4. Dispositif de guidage selon l'une des revendications 1 à 3, **caractérisé en ce que** les champs magnétiques (4) peuvent être produits par application d'une tension électrique aux fils métalliques (2, 3).

5. Dispositif de guidage selon l'une des revendications précédentes, **caractérisé en ce que** les fils métalliques (2, 3) sont disposés l'un à côté de l'autre et parallèlement l'un à l'autre, de préférence torsadés l'un par rapport à l'autre, ou **en ce que** les fils (2, 3) sont disposés d'une manière concentrique l'un de l'autre, au moins l'un des fils (2, 3) étant en position centrale intérieure, et l'autre fil (3, 2) étant disposé en hélice, extérieurement dans la direction radiale, autour du premier.

6. Dispositif de guidage selon la revendication 5, **caractérisé en ce que** le premier fil (2) est disposé en position centrale intérieure, et, autour de la périphérie extérieure du premier fil (2), plusieurs deuxièmes fils (3) sont disposés, en étant de préférence équidistants les uns des autres.

7. Dispositif de guidage selon l'une des revendications précédentes, **caractérisé en ce que** les fils (2, 3) sont configurés de telle sorte que, lors de la production d'un champ magnétique pour créer une attraction réciproque, ils s'appuient en pleine surface l'un contre l'autre.

8. Dispositif de guidage selon l'une des revendications précédentes, **caractérisé en ce que** les champs magnétiques (4) peuvent être produits avec une aimantation permanente, auquel cas de préférence chaque fil métallique (2, 3) est, sur toute sa longueur et dans la direction radiale, soumis en alternance à une polarisation inversée.

9. Dispositif de guidage selon l'une des revendications précédentes, **caractérisé en ce que** le dispositif de commande (8) autorise un contact ou un engrènement réciproque de surfaces de base (6, 10), en regard l'une de l'autre, des fils (2, 3), de préférence sous forme d'une denture (15) et, par introduction d'un fluide sous pression, de préférence d'un liquide ou d'un gaz, dans la fente (16) située entre les fils (2, 3), une séparation l'une de l'autre des surfaces de base (6, 10).

10. Dispositif de guidage selon la revendication 9, **caractérisé en ce que** le contact ou l'engrènement réciproque des surfaces de base en regard l'une de l'autre (6, 10) des fils (2, 3) peut être réalisé par attraction du fluide, de préférence sous l'effet de l'application supplémentaire d'un vide.
